# EUROPÄISCHE PATENTANMELDUNG

(11) **EP 4 470 630 A1**
(43) Veröffentlichungstag der Anmeldung: **04.12.2024**
(21) Anmeldenummer: 23176565.2
(22) Anmeldetag: 31.05.2023
(51) Int. Cl.: A61Q 19/00, A61Q 19/08, A61K 8/9789

(54) **KOSMETISCHE ZUSAMMENSETZUNG ENTHALTEND EINEN EXTRAKT AUS GRANATAPFELSCHALEN**

(71) Anmelder: Weleda AG, 4144 Arlesheim (CH)
(72) Erfinder: IDOUX, Alicia, Bättwil (CH); KAPFER, Hélène, Bettlach (FR)
(74) Vertreter: Simandi, Claus

(57) **Zusammenfassung**

Die Erfindung betrifft eine kosmetische Zusammensetzung enthaltend einen enzymatisch hergestellten Extrakt aus Granatapfelschalen, wobei gezielt sekundäre Inhaltsstoffe angereichert sind. Weiterhin betrifft die Erfindung einen enzymatisch hergestellten Extrakt aus Granatapfelschalen, ein Verfahren zur Herstellung und dessen Verwendung.

## Beschreibung

Die Erfindung betrifft eine kosmetische Zusammensetzung enthaltend einen enzymatisch hergestellten Extrakt aus Granatapfelschalen, wobei gezielt sekundäre Inhaltsstoffe angereichert sind. Weiterhin betrifft die Erfindung einen enzymatisch hergestellten Extrakt aus Granatapfelschalen, ein Verfahren zur Herstellung und dessen Verwendung.

Der Granatapfel (*Punica granatum*) ist eine Pflanzenart aus der Familie der Weiderichgewächse (Lythraceae). Der Granatapfel stammt ursprünglich aus dem östlichen Mittelmeerraum und wird heute in vielen Regionen der Welt angebaut, einschließlich des Nahen Ostens, Nordafrikas, Asiens und Nordamerikas. Er bevorzugt trockene, subtropische und mediterrane Klimazonen. Die Frucht des Granatapfels ist eine Beere, die botanisch als "Scheinbeere" bezeichnet wird. Jeder Granatapfel enthält eine Vielzahl von Samen, die von einer saftigen, durchsichtigen Hülle umgeben sind. Die Hülle ist das essbare Fruchtfleisch, welche von der Granatapfelschale (Perikarp) geschützt wird.

Weleda AG forscht mit modernen und in Verbindung mit anthroposophischen Methoden an vorteilhaften natürlichen Pflanzenstoffen für die Kosmetik.

Die Haut übt als das größte Organ des Menschen zahlreiche lebenswichtige Funktionen aus. Sie schützt z.B. vor Kälte, Hitze, Strahlung, dem Einwirken chemischer Substanzen und Krankheitserregern. Sofern die Haut diese Barrierefunktion nicht mehr ausreichend erfüllt, können lokale Irritationen oder auch ganzkörperliche Beschwerden erfolgen.

Die Haut, insbesondere die Epidermis, ist als Barriereorgan des menschlichen Organismus in besonderem Maße äußeren Einwirkungen unterworfen. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedenen polaren Lipiden werden im Keratinisierungsprozess freigesetzt.

In einem optimalen Zustand der Haut liegt ein ausgewogenes Verhältnis von Hautlipiden und Hautfeuchtigkeit vor. Durch dieses Gleichgewicht werden wichtige Eigenschaften der Haut, wie das Penetrationsvermögen, Wasserbindungsvermögen, Elastizität, Regenerationsfähigkeit oder die Widerstandsfähigkeit gegen Umwelteinflüsse und Noxen unterschiedlichster Art, bestimmt.

Gerade natürliche Pflanzenstoffe haben einen pflegenden und wohltuenden Effekt auf der Haut, insbesondere sekundäre Pflanzeninhaltsstoffe sind von Vorteil, wobei die Granatapfelschale besonders reich an sekundären Pflanzeninhaltsstoffen ist.

Die Granatapfelschale ist die äußere, harte Schicht des Granatapfels (Exocarp genannt), welche hauptsächlich Tannine enthält. Diese Tannine in den Granatapfelschalen sind für den bitteren Geschmack verantwortlich und verleihen der Schale eine gewisse Festigkeit und Widerstandsfähigkeit. Unter der äußeren Schicht befindet sich die mittlere Schicht der Granatapfelschale, die als Mesokarp bezeichnet wird. Das Mesokarp besteht aus einer Mischung aus Zellulosefasern, Lignin und weiteren Tanninen. Diese Kombination verleiht der Schale zusätzliche Festigkeit und Struktur. Die innerste Schicht der Granatapfelschale wird als Endokarp bezeichnet. Sie ist dicker als die äußeren Schichten und besteht hauptsächlich aus weißen Zellulosefasern. Diese Fasern sind sehr zäh und dienen dazu, die Samen des Granatapfels in einzigartiger Weise zu schützen.

Die unterschiedlichen Schichten (Exokarp, Mesokarp und Endokarp) der Granatapfelschale werden auch als Perikarp bezeichnet. Die chemische Zusammensetzung der Granatapfelschalen enthält charakteristisch in hohen Anteilen Flavonoide, Phenolsäuren, Polyphenole und Anthocyane, welche für die vorteilhaften antioxidativen Eigenschaften des Granatapfels verantwortlich sind.

Erfindungsgemäß sollen die wertvollen Inhaltsstoffe der Granatapfelschalen für eine kosmetische Formulierung in geeigneter Weise bereitgestellt werden. Hierzu ist erforderlich, dass die Granatapfelschalen aufgeschlossen werden. Besonders schonend kann ein solcher Aufschluss statt nasschemisch oder mit physikalischen Methoden durch einen enzymatischen Aufschluss sehr effizient erfolgen.

Im Stand der Technik sind Enzymcocktails bzw. Enzymmischungen beschrieben, die für den Aufschluss von Granatapfelschalen geeignet sein sollen.

EP 1901756 B1 offenbart die enzymatische Behandlung des Fruchtfleisches samt Perikarp von Granatäpfeln.

EP 3235386 A1 beschreibt die enzymatische Herstellung eines Fruchtschalenextraktes mit Zugabe von Zitronensäure zur Färbung des Extrakts.

WO 2000056177 A2 beschreibt die enzymatische Herstellung eines Granatapfelschalenextrakts aus wässriger Lösung. Weiterhin wird offenbart, dass solche Schalenextrakte aufgrund des hohen Polyphenolgehaltes antioxidativ sind.

Der vorliegenden Erfindung liegt daher die Aufgabe zugrunde, sowohl eine kosmetische Zusammensetzung zur topischen Verwendung aus einem Granatapfelschalenextrakt bereitzustellen als auch ein Verfahren zur Herstellung eines solchen Granatapfelschalenextrakts.

Überraschenderweise konnten die Erfinder feststellen, dass bei Zugabe von Protease zu einem Enzymcocktail der Polyphenolgehalt in einem Extrakt aus Granatapfelschalen vorteilhaft *spezifisch* erhöht werden kann, und folglich eine spezifische Anreicherung von Polyphenolen im Extrakt erfolgt (siehe Beispiele).

Die Aufgabe wird daher durch ein Verfahren zur Herstellung eines Granatapfelschalenextrakts gelöst, wobei folgende Schritte durchgeführt werden:
a.) Bereitstellung von Granatapfelschalen,
b.) Zerkleinern,
c.) Versetzen mit einer wässrigen oder wässrig-alkoholischen Lösung, wobei der Alkohol ein C1-C5 Alkohol sein kann,
d.) Versetzen in der Wärme bei 30 - 70 Grad Celsius mit mindestens einem oder mehreren Enzymen, wobei mindestens ein Enzym eine Protease ist,
e.) anschließend Zentrifugation und/oder Filtration.

Die erfindungsgemäße Protease kann der Enzymklasse EC 3.4 entnommen werden, solche wie Aminopeptidasen, Dipeptidasen, Dipeptidyl-Peptidasen, Peptidyl-Dipeptidasen, Peptidyl-Dipeptidasen, Serin-Carboxypeptidasen, Metallocarboxypeptidasen, Cystein-Carboxypeptidasen, Omegapeptidasen, Serin-Endopeptidasen, Cystein-Endopeptidasen, Aspartat-Endopeptidasen, Metalloendopeptidasen, Threonin-Endopeptidasen.

Weiterhin können neben einer Protease folgende Enzyme eingesetzt werden, wie Cellulasen, Hemicellulasen oder Pectinasen. Diese Enzyme dienen vor allem zum Aufschluss von (Hemi-)Cellulose, Lignin, Pektinen u.a.

Weiterhin kann erfindungsgemäß ein Emzymcocktail eingesetzt werden, wobei neben mindestens einer Protease mindestens eine Cellulase, Hemicellulase oder Pectinase eingesetzt wird. Die Cellulasen, Hemicellulase oder Pectinase können der EC-Klasse 3.2 entnommen werden.

Der Anteil einer Protease an diesem Enzymcocktail beträgt zumindest 1 Gew. %, 2 Gew. %, 5 Gew. % oder 10 Gew. % oder mehr.

In einer weiteren bevorzugten Ausführungsform kann die Lösung in Schritt c.) und / oder Schritt d.) mindestens über eine halbe Stunde oder über Stunden oder Tage stehen gelassen werden, bevor jeweils der nachfolgende Schritt d.) oder die Zentrifugation und / oder Filtration e.) erfolgt.

Weiterhin kann nach Zentrifugation und / oder Filtration ein erneutes Versetzen erfolgen. Das Stehenlassen der Lösung kann eine Mazeration sein, ebenfalls eine Bewegungsmazeration, wobei die Bewegung vorzugsweise mit Hilfe von Rühren bewirkt wird.

Die Filtration gemäß Schritt e.) erfolgt vorzugsweise mit einem oder mehrere Sieben, wobei die Sieböffnungen vorzugsweise 2-300 µm betragen. Dies erlaubt das Zurückhalten von Feststoffen, so dass ein homogener und klarer Extrakt aus Granatapfelschalen erhalten werden kann.

Weiterhin erfolgt die Zentrifugation gemäß Schritt e.) bei vorzugsweise 1.000 - 2.000 U /min.

In einer besonders bevorzugten Ausführungsform beträgt der Anteil an Alkohol, insbesondere Ethanol max. 70 % (m/m) im alkoholischen-wässrigen, insbesondere ethanolisch-wässrigen Lösung, vorzugsweise 70 % (70:30) (m/m) und weniger, jedoch vorzugsweise mehr als 10 % (m/m), insbesondere 30 % (m/m).

Weiterhin können C1-C5 Alkohole, wie Methanol (C1), Ethanol (C2), Propanol (C3), Butanol (C4), Pentanol (C5) verwendet werden.

Ebenfalls können alkoholisch-wässrige Auszüge mit mehrwertigen Alkoholen erfolgen, insbesondere zweiwertige und dreiwertige Alkohole, insbesondere solche wie Glykol, 1,2-Propandiol, Glycerin, 1,2-Pentandiol oder andere Polyole.

Bei Schritt c.) wird die Granatapfelschale eine bestimmte Zeit in eine Flüssigkeit, insbesondere in der bestehenden Flüssigkeit, jedoch vorzugsweise in eine wässrige oder wässrig-alkoholischen Flüssigkeit (supra) eingelegt, wobei das Mazerat (Flüssigkeit) den Granatapfelschalenextrakt umfasst.

In einer bevorzugten Ausführungsform wird im Schritt b.) das Pflanzenmaterial, und zwar Granatapfelschalen frisch, getrocknet oder tiefgefroren und zerkleinert. Blüten und Fruchtfleisch sind nicht als Pflanzenmaterial zu berücksichtigen und auszunehmen.

In einer weiteren bevorzugten Ausführungsform kann die Wärmebehandlung gemäß Schritt d.) unter Rühren erfolgen.

Weiterhin ist bevorzugt, dass die Wärmebehandlung gemäß Schritt d.) bei 30 - 70 Grad Celsius, insbesondere bei 40 - 60 Grad Celsius, insbesondere 50 Grad Celsius erfolgt.

Weiterhin ist bevorzugt, dass die Wärmebehandlung gemäß Schritt d.) innerhalb von 3 h, 4h oder 5 h erfolgt.

Das erfindungsgemäße Verfahren erlaubt die vorteilhafte spezifische Anreicherung von Poly(hydroxy)phenolen. Es handelt sich hierbei um Extrakt-eigene Polyphenole, die aufgrund des erfindungsgemäßen Verfahrens aus dem Pflanzenstoff ausgelöst werden, siehe Beispiele.

Daher betrifft die Erfindung ebenfalls einen Granatapfelschalenextrakt erhältlich aus dem erfindungsgemäßen Verfahren, wobei der Polyphenolgehalt spezifisch erhöht ist.

Polyphenole können im Granatapfel anhand der (Leit-)Substanzen Punicaline, nämlich Punicalin A (CAS 157201-76-8) und Punicalin B (CAS 157201-77-9) und Punicalagine, nämlich Punicalagin A (CAS 130518-17-1) und Punicalagin B (CAS 30608-10-5) identifiziert werden.

Daher betrifft die Erfindung insbesondere ein solcher Granatapfelschalenextrakt, der mindestens 0,8 Gew. % oder mindestens 1,0 Gew. % oder mindestens 1,2 Gew. % Punicaline, nämlich Punicalin A (CAS 157201-76-8) und Punicalin B (CAS 157201-77-9) und Punicalagine, nämlich Punicalagin A (CAS 130518-17-1) und Punicalagin B (CAS 30608-10-5) im erhaltenen Flüssigextrakt enthalten, siehe Beispiele.

Besonders bevorzugt ist, dass der erhaltene Flüssigextrakt mit einer 10 - 20 % igen (w/w), insbesondere 15 % -igen (w/w) Alkohollösung, insbesondere 1,2-Pentandiol-Lösung stabilisiert ist. Weiterhin kann der erhaltene flüssige Extrakt getrocknet werden.

Der erhaltene Extrakt kann in einem Kosmetikum verwendet werden.

In einer weiteren Ausführungsform kann die erfindungsgemäße kosmetische oder dermatologische Zubereitung zur topischen Verwendung in wasserfreier sowie flüssiger und fester Form, in Form eines Gels, eines Sprays, einer Creme, eines Serums, einer Emulsion oder einer Lotion erfolgen.

Emulgatoren können vorteilhaft gewählt werden aus der Gruppe der nichtionischen, anionischen, kationischen oder amphoteren Emulgatoren.

Erfindungsgemäß bevorzugt sind natürlich vorkommende Emulgatoren, zu denen beispielsweise Bienenwachs, Wollwachs, Lecithin und Sterole u.a. gehören, die ebenfalls bei der Herstellung einer erfindungsgemäßen Zusammensetzung eingesetzt werden können.

Die erfindungsgemäßen kosmetischen Zusammensetzungen können weiterhin kosmetische Hilfsstoffe enthalten, wie sie üblicherweise in solchen Zubereitungen verwendet werden, z. B. Antioxidantien, Radikalfänger, Konservierungsmittel, Bakterizide, Parfüme, Farbstoffe, Pigmente, die eine färbende Wirkung haben, Verdickungsmittel, oberflächenaktive Substanzen, weichmachende, oder andere übliche Bestandteile einer kosmetischen Formulierung wie Alkohole, Polyole, Polymere, Schaumstabilisatoren, Elektrolyte, organische Lösemittel.

In einer weiteren besonderen Ausführungsform besteht die erfindungsgemäße Zusammensetzung vorzugsweise aus natürlich vorkommenden oder naturnahen, respektive naturidentischen Inhaltsstoffen.

Nachfolgende Ausführungsbeispiele und Figuren dienen zur Erläuterung der Erfindung, ohne die Erfindung auf diese Beispiele zu beschränken.

### Beispiel 1:

In diesem Beispiel werden zwei Granatapfelschalenextrakte Ansätze aus gleichen Ausgangsmaterial mit zwei unterschiedlichen Enzymcocktails, und zwar mit und ohne Protease hergestellt. Der jeweilige Enzymcocktail enthält jeweils in gleichen Mengen Cellulasen, Hemicellulasen und Pectinasen.

Als Vergleichsmerkmal werden mittels HPLC die Gehalte von den für die Granatapfelschalen spezifischen Polyphenolen Punicaline und Punicalagine bestimmt und gegeneinandergestellt.

HPLC-Methode zur quantitativen Bestimmung von Punicaline und Punicalagine in flüssigem Granatapfelschalen-Extrakt:
Referenzen:
Punicalagin A+B, Phytolab, Art. Nr. 80524
Punicalin A+B, Phytolab, Art. Nr. 83532

| | | |
|---|---|---|
| Säule: | RP-18, 5pm, 250mm Länge, 4mm Durchmesser | |
| Mobile Phase: | | A: Wasser mit 0.1% Trifluoressigsäure |
| | | B: Acetonitril |
| Gradient: | | |

| Zeit (Minuten) | Lösemittel A (%) | Lösemittel B (%) |
|---|---|---|
| 0 | 100 | 0 |
| 25 | 88 | 12 |
| 45 | 70 | 30 |
| 45.1 | 100 | 0 |
| 50 | stop | stop |

| | |
|---|---|
| Ofentemperatur: | 40°C |
| Fluss: | 1ml/min |
| Injektionsvolumen: | 10µl |
| Detektion: | 260nm |

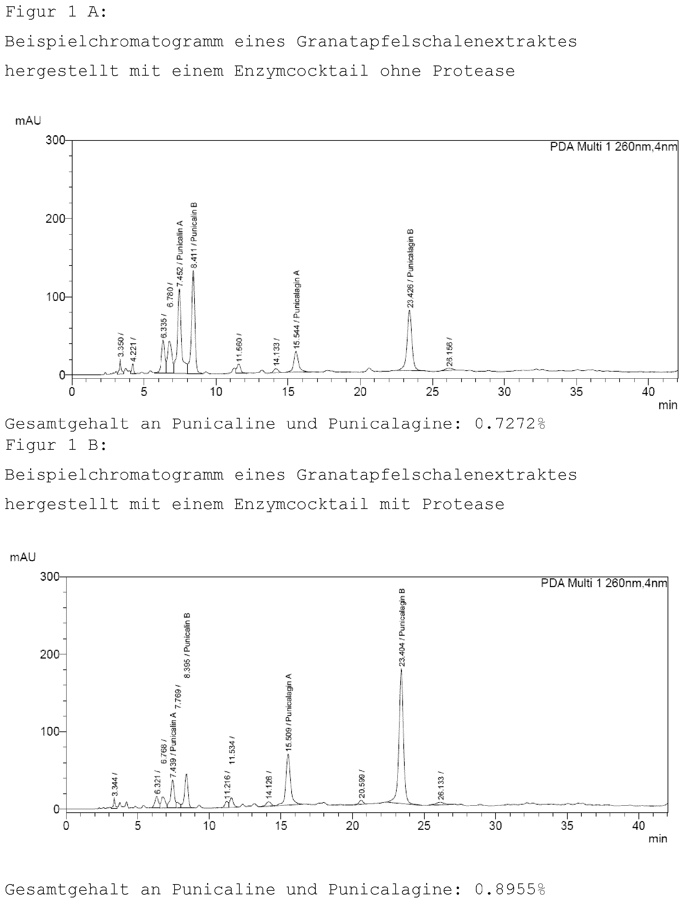

Die Vergleichschromatogramme von einem flüssigen erfindungsgemäßen Granatapfelschalen-Extrakt sind in Figur 1A (Beispielchromatogramm eines Granatapfelschalenextraktes hergestellt mit dem Enzymcocktail ohne Protease) und in Figur 1B (Beispielchromatogramm eines Granatapfelschalenextraktes hergestellt mit dem Enzymcocktail mit Protease) dargestellt.

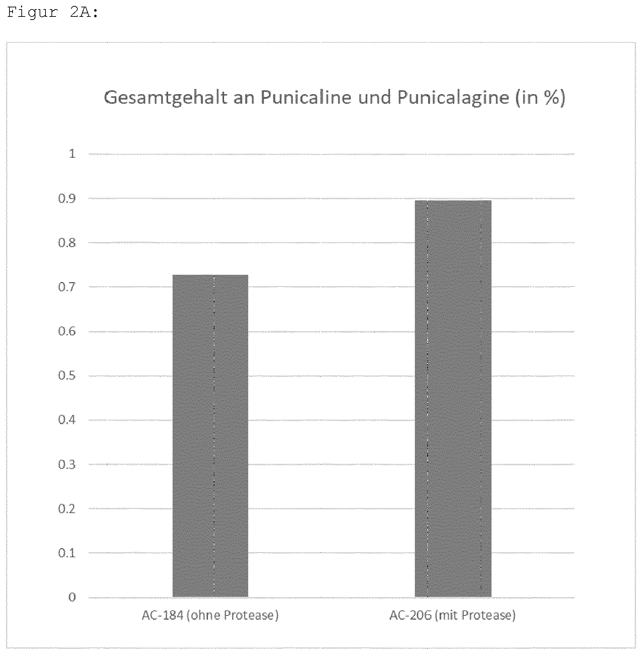

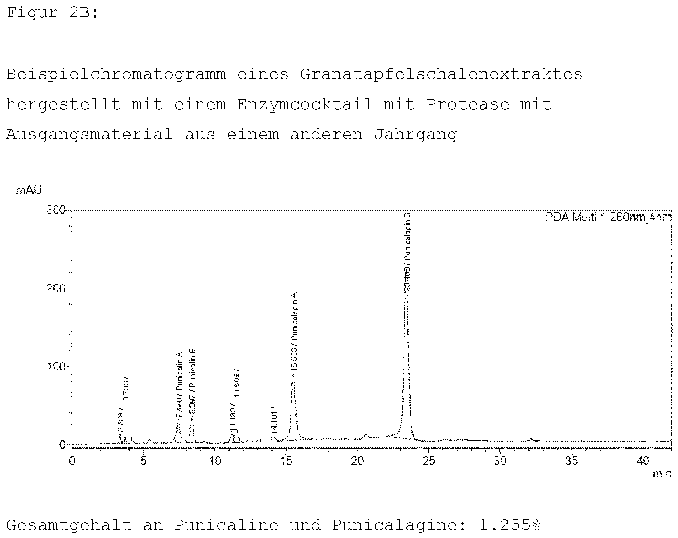

Der Balkendiagramm in Figur 2A stellt jeweils den Gesamtgehalt an Punicaline und Punicalagine dar. Dieser wurde anhand der Flächen der beiden jeweiligen Peaks (2 für Punicalin und 2 für Punicalagin) im Chromatogramm bei der gegebenen Wellenlänge anhand von der Kalibrierung mit den jeweiligen Referenzen berechnet.

Ein Unterschied in den Gehalten an den Polyphenolen ist zwischen beiden Proben zugunsten des Herstellverfahren mit dem Enzymcocktail mit Protease gut sichtbar.

Ferner ist zu erkennen, dass die Polyphenole vorteilhaft spezifisch angereichert werden (siehe Verhältnisse der Peaks).

## Patentansprüche

1. Verfahren zur Herstellung eines Granatapfelschalenextrakts mit folgenden Schritten:
a.) Bereitstellung von Granatapfelschalen,
b.) Zerkleinern,
c.) Versetzen mit einer wässrigen oder wässrig-alkoholischen Lösung, wobei der Alkohol ein C1-C5 Alkohol sein kann,
d.) Versetzen in der Wärme bei 30 - 70 Grad Celsius mit mindestens einem oder mehreren Enzymen, wobei mindestens ein Enzym eine Protease ist,
e.) anschließend Zentrifugation und/oder Filtration.

2. Verfahren zur Herstellung eines Granatapfelschalenextrakts nach Anspruch 1, wobei neben der Protease in Schritt d.) mindestens eine Cellulase, Hemicellulase oder Pectinase umfasst ist.

3. Verfahren zur Herstellung eines Granatapfelschalenextrakts nach einem der vorhergehenden Ansprüche 1 bis 2, wobei der Alkohol ein C1-C5 Alkohol ist.

4. Verfahren zur Herstellung eines Granatapfelschalenextrakts nach einem der vorhergehenden Ansprüche 1 bis 3, wobei der Alkohol ein mehrwertiger Alkohol ist, insbesondere ausgewählt aus der Gruppe Glykol, 1,2-Propandiol, Glycerin, 1,2 Pentandiol oder Polyol.

5. Verfahren zur Herstellung eines Granatapfelschalenextrakts nach einem der vorhergehenden Ansprüche 1 bis 4, **dadurch gekennzeichnet, dass** das Filtern in Schritt e.) mit ein oder mehreren Sieben erfolgt, wobei die Sieböffnungen 2-300 µm betragen.

6. Flüssiger Granatapfelschalenextrakt erhältlich nach einem der vorhergehenden Ansprüche 1 bis 5, wobei mindestens 0,8 Gew. % oder mindestens 1,0 Gew. % oder mindestens 1,2 Gew. % Punicaline und Punicalagine enthalten sind.

7. Trockener Granatapfelschalenextrakt erhältlich nach Trocknen eines flüssigen Granatapfelschalenextrakts nach Anspruch 6.

8. Kosmetikum enthaltend einen Granatapfelschalenextrakt nach Anspruch 6 oder 7.

9. Kosmetikum nach Anspruch 8 in Form eines Gels, eines Sprays, einer Creme oder einer Lotion, einer Emulsion, eines Serums oder in einer festen, flüssigen oder wasserfreien Form.
